# EUROPÄISCHE PATENTSCHRIFT

(11) **EP 3 577 071 B1**
(45) Veröffentlichungstag und Bekanntmachung des Hinweises auf die Patenterteilung: **29.09.2021**
(21) Anmeldenummer: 18705357.4
(22) Anmeldetag: 08.02.2018
(51) Int. Cl.: C01B 33/02, C01B 33/107

(54) **VERFAHREN ZUR KLASSIFIZIERUNG VON METALLURGISCHEM SILICIUM**
PROCESS FOR CLASSIFICATION OF METALLURGICAL SILICON
PROCÉDÉ DE CLASSIFICATION DE SILICIUM MÉTALLURGIQUE

(43) Veröffentlichungstag der Anmeldung: 11.12.2019
(73) Patentinhaber: Wacker Chemie AG, 81737 München (DE)
(72) Erfinder: RIMBÖCK, Karl-Heinz, 84431 Heldenstein (DE); PÄTZOLD, Uwe, 84489 Burghausen (DE); TRAUNSPURGER, Gerhard, 83329 Waging am See (DE)
(74) Vertreter: Belz, Ferdinand
(86) Internationale Anmeldenummer: PCT/EP2018/053210
(87) Internationale Veröffentlichungsnummer: WO 2019/154502

(56) Entgegenhaltungen:
- EP-A1- 0 260 424
- WO-A1-2012/109459
- DE-A1-102015 210 762
- US-A1- 2007 248 521
- US-A1- 2014 050 648
- US-A1- 2018 008 381

## Beschreibung

Die Erfindung betrifft ein Verfahren zur Klassifizierung von metallurgischem Silicium, das Verunreinigungen aus Kohlenstoff und/oder kohlenstoffhaltigen Verbindungen enthält, sowie die Verwendung von metallurgischem Silicium mit einem Gesamtkohlenstoffgehalt von bis zu 2500 ppmw zur Herstellung von Chlorsilanen.

Metallurgisches Silicium (Si_{mg}, "metallurgical grade" Silicium) oder auch Rohsilicium wird im industriellen Maßstab durch Reduktion von Siliciumdioxid mit Kohlenstoff in einem Lichtbogenofen bei einer Temperatur von etwa 2000°C hergestellt. Die Reinheit von Si_{mg} beträgt üblicherweise etwa 98-99%.

Si_{mg} ist Ausgangsstoff für die Herstellung von Methylchlorsilanen nach dem Müller-Rochow-Verfahren. Methylchlorsilane dienen insbesondere zur Herstellung von Silikonen.

Des Weiteren findet es als Ausgangsstoff Anwendung in der Photovoltaik. Dafür muss das Si_{mg} gereinigt und in Solarsilicium (Si_{sg}, "solar grade" Silicium), das in der Regel Verunreinigungen von weniger als 0,01% aufweist, umgewandelt werden. Dazu wird es beispielsweise mit gasförmigem Chlorwasserstoff bei 300 bis 700°C in einem Wirbelschichtreaktor zu Chlorsilanen, insbesondere Trichlorsilan (TCS, HSiCl₃), umgesetzt. Es folgen Destillationsschritte, um die Chlorsilane weiter zu reinigen. Die so erhaltenen Chlorsilane dienen dann als Ausgangsstoff für die Herstellung von polykristallinem Silicium (Poly-Si).

Poly-Si kann nach dem Siemens-Verfahren in Form von Stäben erzeugt werden, wobei elementares Silicium in einem Reaktor an erhitzten Filamentstäben aus der Gasphase abgeschieden wird.

Als Prozessgas wird meist ein Gemisch aus TCS und Wasserstoff eingesetzt. Alternativ kann Poly-Si in Form eines Granulats hergestellt werden. Dabei werden Siliciumpartikel mittels einer Gasströmung in einem Wirbelschichtreaktor fluidisiert und erhitzt. Durch Zugabe eines siliciumhaltigen Reaktionsgases, z.B. TCS, erfolgt eine Abscheidung von elementarem Silicium an den heißen Partikeloberflächen, wodurch die Partikel in ihrem Durchmesser wachsen.

Poly-Si wiederum ist Ausgangsmaterial bei der Herstellung von multikristallinem Si_{sg}, z.B. nach dem Blockgussverfahren. Aus dem Si_{sg} können dann Solarzellen gefertigt werden.

Die bereits erwähnte Umwandlung von Si_{mg} in Chlorsilane, z.B. TCS, kann durch zwei Verfahren erfolgen, denen folgende Reaktionsgleichungen zugrunde liegen (vgl. WO2010/028878A1 und WO2016/198264A1) :

(1) Si + 3HCl --> SiHCl₃ + H₂ + Nebenprodukte

(2) Si + 3SiCl₄ + 2H₂ --> 4SiHCl₃ + Nebenprodukte

Als Nebenprodukte können weitere Halogensilane anfallen, beispielsweise Monochlorsilan (H₃siCl), Dichlorsilan (H₂SiCl₂), Siliciumtetrachlorid (STC, SiCl₄) sowie Di- und Oligosilane. Ferner können Verunreinigungen wie Kohlenwasserstoffe, Organochlorsilane und Metallchloride Bestandteil der Nebenprodukte sein. Um hochreines TCS zu erzeugen, erfolgt daher anschließend eine Destillation.

Bei der Hydrochlorierung gemäß Reaktion (1) können aus Si_{mg} unter Zusatz von Chlorwasserstoff (HCl) Chlorsilane in einem Wirbelschichtreaktor bei 300 bis 700°C hergestellt werden, wobei die Reaktion exotherm verläuft. Dabei erhält man in der Regel TCS und STC als Hauptprodukte.

Die Niedertemperaturkonvertierung (NTK) gemäß Reaktion (2) wird in Gegenwart eines Katalysators (z.B. Cu) durchgeführt. Die NTK kann in einem Wirbelschichtreaktor in Anwesenheit von Si_{mg} bei Temperaturen zwischen 400°C und 700°C erfolgen.

Es ist bekannt, dass Kohlenstoff oder kohlenstoffhaltige Verunreinigungen bei der Herstellung von Poly-Si zu einer verminderten Qualität (negative Einflüsse auf die elektrischen Eigenschaften) des Poly-Si selbst und dessen Folgeprodukten führen. Beispielsweise können kohlenstoffhaltige Verunreinigungen Defekte im Kristallgitter von aus Poly-Si hergestellten Siliciumeinkristallen führen und/oder zu einer beschleunigten Bildung von Sauerstoff-Präzipitaten.

Es ist daher wichtig, den Kohlenstoffgehalt im gesamten Herstellungsverlauf von Poly-Si so gering wie möglich zu halten.

Die kohlenstoffhaltigen Verunreinigungen können beispielsweise aus kohlenstoffhaltigen Bauteilen der Siemens- oder Wirbelschichtreaktoren in den Prozess eingetragen werden. Eine weitere Kohlenstoffquelle stellen die im Herstellungsverlauf verwendeten Reaktanden dar, die Kohlenstoff oder kohlenstoffhaltige Verbindungen als Verunreinigungen enthalten können.

Eine Hauptursache für kohlenstoffhaltigen Verunreinigungen sind die bei der Herstellung von Chlorsilanen verwendeten Reaktanden Si_{mg}, Wasserstoff und Chlorwasserstoff (HCl). Beispielsweise können die Reaktionsgase Methan, Ethan, Kohlenmonoxid und Kohlendioxid (CO₂) enthalten. Diese Verbindungen können insbesondere aus zurückgewonnenem Wasserstoff und/oder HCl stammen.

Si_{mg}, das in der Regel partikulär vorliegt und eine Reinheit von 98% bis 99% aufweist, kann neben Kohlenstoff folgende Elemente enthalten: Fe, Ca, Al, Ti, Cu, Mn, Cr, V, Ni, Co, W, Mo, As, Sb, Bi, S, Se, Te, Zr, Ge, Sn, Pb, Zn, Cd, Sr, Ba, Y, Mg, B, P, O und Cl. Einige dieser Elemente, bspw. Cu, Al und Fe, wirken katalytisch bei der Hydrochlorierung und NTK, weshalb ihre Anwesenheit durchaus erwünscht sein kann.

Si_{mg} weist typischerweise Kohlenstoff in einer Menge von 1 bis 2500 ppmw auf. In der Regel liegt der Kohlenstoffgehalt über 300 ppmw. Der Kohlenstoff kann dabei gebunden als Siliciumcarbid (SiC) vorliegen (anorganischer Kohlenstoff). Ferner kann er auf der Oberfläche des Si_{mg} in Form von organischen Verbindungen (z.B. Hydrauliköle) und elementar in seinen allotropen Formen vorliegen. Eingetragen wird der Kohlenstoff insbesondere während des Herstellungsprozesses von Si_{mg} (z.B. durch Elektroden und Reduktionsmittel), bei Zerkleinerungsprozessen (durch Mahl-/Brecheranlagen) sowie bei Klassierungsprozessen (durch Sieb-/Sichteranlagen).

Die EP 0 260 424 A1 beschreibt ein Verfahren zur Herstellung von kohlenstoffarmem Silicium, wobei Si_{mg} mit einem Reinheitsgrad von ca. 98% und einem Kohlenstoffgehalt von 700 bis 1500 ppma (300-643 ppmw) als Ausgangsmaterial verwendet wird.

Die US 2014/0050648 A1 beschreibt ein Verfahren zur Herstellung von Chlorsilanen, wobei neben ultrareinem und ultrafeinem Silicium mit einer Reinheit von mehr als 99,9999% Si_{mg} mit einer Reinheit von weniger als 99,9% eingesetzt wird.

Aus der WO 2012/109459 A1 ist ein Verfahren zur Herstellung von Chlorsilanen beschrieben, wobei als Rohstoff Si_{mg} eingesetzt wird, das nach einer Aufreinigung einen Kohlenstoffgehalt von weniger als 100 ppm aufweist.

Die US 2007/0248521 A1 offenbart ein Verfahren zur Herstellung von Chlorsilanen, bei dem Si_{mg} mit einem Kohlenstoffanteil von 50 bis 1500 ppm eingesetzt wird.

Der Großteil des in Si_{mg} enthaltenen Kohlenstoffs liegt generell in Form von SiC vor und verhält sich scheinbar inert bei der Hydrochlorierung und NTK (Hesse K., Pätzold U.: Survey over the TCS process, Silicon for the Chemical Industry VIII 2006, 157-166). Ein kleiner Teil des Kohlenstoffs reagiert bei den o.g. Verfahren zu Methylchlorsilanen. Organische Verunreinigungen können unter den Bedingungen der Hydrochlorierung und NTK zu kurzkettigen Kohlenwasserstoffen und Chlorkohlenwasserstoffen reagieren. Einige dieser Nebenprodukte weisen einen ähnlichen Siedepunkt wie die zu erhaltenden Chlorsilane, insbesondere TCS, auf und lassen sich deshalb nur schwer mittels Destillation abtrennen (Kohno H., Kuroko T., Itoh H., Quality requirements for silicon metal in polysilicon production, Silicon for the Chemical Industry II, 1994, 165-170).

Besonders Methyldichlorsilan (MDCS) und Kohlenwasserstoffe der C5-Fraktion (iso-Pentan, Pent-1-en, Pent-2-en) lassen sich destillativ nur aufwendig abtrennen. Üblicherweise erfolgt ein Trennverfahren in einem System, bestehend aus mehreren Destillationskolonnen. Dies führt einerseits zu hohen Investitionskosten und andererseits zu einem hohen Energieverbrauch. Ein weiteres Problem stellt die Entsorgung der anfallenden Chlorsilan-Ströme dar, in denen die Nebenprodukte angereichert sind.

Ein Verfahren, zur Entfernung von MDCS, das auf Adsorption an einer festen Phase beruht, ist aus der JP2004149351A bekannt. Ferner beschreibt die EP 2 957 543 A1 ein Verfahren, das auf einer Umwandlung von schwer mittels Destillation abtrennbarer MDCS zu leichter abtrennbaren Methyltrichlorsilan (MTCS) beruht. Allerdings beinhalten diese Ansätze weitere zusätzliche und aufwändige Prozessschritte und vermindern daher den Energieverbrauch und die Investitionskosten nur unwesentlich.

Rong et al. beschreiben ein Verfahren, das das Ziel hat, zwischen verschiedenen Kohlenstoffspezies auf bzw. in Si_{mg} zu unterscheiden (Rong et al., Analytical method to measure different forms of carbon in silicon, Silicon for the Chemical and Solar Industry XI, 2012, 145-156). Zur Bestimmung der Kohlenstoffspezies wird einerseits der mit O₂ reagierende Gesamtkohlenstoffgehalt des Si_{mg} mit einem Verbrennungsautomat LECO C-200 untersucht. Andererseits wird der Gehalt an freiem Kohlenstoff mit einem Verbrennungsautomat LECO RC-612 ermittelt, wobei es sich dabei definitionsgemäß um den Anteil an Kohlenstoff handelt, der bei Temperaturen von 950°C und 600°C mit O₂ reagiert. Es wird ausgeführt, dass es bei der TCS-Synthese (Hydrochlorierung) ausgehend von Si_{mg} zur Bildung kohlenstoffhaltiger Nebenprodukte kommen kann. Allerdings sei der weitaus größte Teil der Kohlenstoffspezies, mit welchen das Si_{mg} verunreinigt ist, unter den Reaktionsbedingungen der Hydrochlorierung inert, führe also zu keiner Bildung von Nebenprodukten. Ein Zusammenhang zwischen den analytisch erfassten Verunreinigungen und der Bildung der Nebenprodukte wird nicht hergestellt. Problematisch seien ferner die gewählten Temperaturbereiche, die zu einer Überbewertung des freien Kohlenstoffs führten.

Die Verwendung eines LECO RC-612 ist auch in der US 2018/0008381 A1 beschrieben.

Die der vorliegenden Erfindung zugrundeliegende Aufgabe bestand darin, den Anteil unerwünschter Nebenprodukte bei der Chlorsilanherstellung zu verringern und damit den Kosten- und Energieaufwand für die Reinigung der Chlorsilane zu senken.

Diese Aufgabe wird gelöst durch ein Verfahren zur Klassifizierung von Si_{mg}, das Verunreinigungen aus Kohlenstoff und/oder kohlenstoffhaltigen Verbindungen enthält, und die folgenden Schritte umfasst:
a) Bestimmen eines freien Kohlenstoffanteils, der bis zu einer Temperatur von 700°C mit O₂ reagiert, und
b) Zuordnen und/oder Bereitstellen von Si_{mg}, bei dem der freie Kohlenstoffanteil kleiner als oder gleich 150 ppmw ist, für ein Verfahren zur Herstellung von Chlorsilanen und/oder Zuordnen und/oder Bereitstellen von Si_{mg}, bei dem der freie Kohlenstoffanteil größer als 150 ppmw ist, für ein Verfahren zur Herstellung von Methylchlorsilanen.

Vorzugsweise wird Si_{mg}, bei dem der freie Kohlenstoffanteil ≤ 80 ppmw, bevorzugt ≤ 30 ppmw, besonders bevorzugt ≤ 10 ppmw, beträgt, dem Verfahren zur Herstellung von Chlorsilanen zugeordnet und/oder Si_{mg}, bei dem der freie Kohlenstoffanteil > 80 ppmw, bevorzugt > 30 ppmw, besonders bevorzugt > 10 ppmw, beträgt, dem Verfahren zur Herstellung von Methylchlorsilanen zugeordnet.

Unter "freiem Kohlenstoff" soll im Sinne der vorliegenden Erfindung der Kohlenstoffanteil des zu klassifizierenden Si_{mg} verstanden werden, der bis zu einer Temperatur von 700°C mit O₂ reagiert, vorzugsweise unter Verwendung eines Verbrennungsautomaten LECO RC-612 (vgl. auch DIN 19539). Bei freiem Kohlenstoff handelt es sich üblicherweise um organischen Kohlenstoff (z.B. Öle, Fette).

Unter "Oberflächenkohlenstoff" soll dagegen der gesamte auf der Oberfläche des Si_{mg} vorhandene Kohlenstoff verstanden werden. Umfasst ist davon sowohl der freie Kohlenstoff als auch der oberhalb von 700°C mit O₂ reagierende Kohlenstoff, bei dem es sich hauptsächlich um anorganischen Kohlenstoff, z.B. SiC, handelt.

Der Begriff "Gesamtkohlenstoff" wiederum schließt neben dem freien Kohlenstoff und dem Oberflächenkohlenstoff auch den sich im Inneren des Si_{mg} befindlichen Kohlenstoff (Bulk-Kohlenstoff) ein.

Es wurde gefunden, dass sich der freie Kohlenstoffanteil, der insbesondere in einem Temperaturbereich von 100 bis 700°C mit O₂ reagiert, unter den Reaktionsbedingungen der Chlorsilanherstellung (insbesondere durch NTK und Hydrochlorierung) nicht inert verhält, sondern zur Bildung von Nebenprodukten führt. Diese können von den gewünschten Chlorsilanen, insbesondere TCS, nur schwer abgetrennt werden. Dagegen verhält sich der anorganische Kohlenstoff, der oberhalb von 700°C mit O₂ reagiert, beim Verfahren zur Chlorsilanherstellung inert. Ferner hat sich gezeigt, dass sich der freie Kohlenstoffanteil proportional zu den bei den Verfahren zur Herstellung von Chlorsilanen gebildeten Mengen an kohlenstoffhaltigen Nebenprodukten verhält, so dass anhand des freien Kohlenstoffgehalts eine qualitative Einteilung des Si_{mg} erfolgen kann. Beispielsweise erhält Si_{mg} mit einem freien Kohlenstoffanteil von weniger als oder gleich 10 ppmw die Qualitätsstufe 1, da nur eine geringe Menge an Nebenprodukten bei der Chlorsilanherstellung zu erwarten ist (vgl. Tabelle 4).

Alternativ oder zusätzlich zu einer statischen Bestimmung des freien Kohlenstoffanteils bei 700°C gemäß Schritt a) kann die Bestimmung auch temperaturfraktioniert für mindestens einen der Temperaturbereiche von 360 bis 400°C, von 480° bis 550°C und von 610 bis 650°C erfolgen. Dabei wird der freie Kohlenstoffanteil, der in dem jeweiligen Temperaturbereich bestimmt wird, mit einer Menge von mindestens einem für den jeweiligen Temperaturbereich charakteristischen Nebenprodukt, das beim Verfahren zur Herstellung von Chlorsilanen entsteht, korreliert. In Abhängigkeit dieser Korrelation kann das Si_{mg} dann hinsichtlich seiner Qualität bewertet werden. Um den freien Kohlenstoffgehalt der Temperaturbereiche abzubilden, wird vorzugsweise ein Temperaturgradient zwischen 100 und 700°C durchlaufen (Temperaturrampe, dynamische Bestimmung) und die ermittelte Menge an freiem Kohlenstoff gegen die Temperatur aufgetragen (Thermogramm).

Es wurde erkannt, dass von der in jedem der drei Temperaturbereiche ermittelten freien Kohlenstoffmenge jeweils auf die Menge von zumindest einem während der Chlorsilanherstellung entstehenden Nebenprodukt geschlossen werden kann. Jedem Temperaturbereich kann mindestens ein Nebenprodukt zugeordnet werden, dessen Konzentration umso höher ist, je höher der ermittelte freie Kohlenstoffanteil in dem entsprechenden Temperaturbereich ist. Die Korrelation lässt sich insbesondere auf Basis von Vergleichs- bzw. Referenzdaten durchführen.

Der im Temperaturbereich von 360 bis 400°C bestimmte freie Kohlenstoffanteil wird mit einer Menge an iso-Pentan, der im Temperaturbereich von 480 bis 550°C bestimmte Anteil mit einer Menge an MTCS und der im Temperaturbereich von 610 bis 650°C bestimmte Anteil mit einer Menge an MDCS korreliert.

Diese drei Nebenprodukte sind typische bei der Herstellung von Chlorsilanen auftretende Verbindungen, die jeweils einen ähnlichen Siedepunkt wie die gewünschten Produkte, insbesondere TCS, aufweisen und daher nur mit einem hohen Aufwand abgetrennt werden können.

In Abhängigkeit der Korrelation kann das Si_{mg} dann gegebenenfalls auch unterschiedlichen Verfahren zur Herstellung von Chlorsilanen zugeordnet werden. Diese Zuordnung kann insbesondere auf den bevorzugten Reaktionstemperaturen beruhen, bei welchen beispielsweise die NTK und die Hydrochlorierung durchgeführt werden. Ist z.B. ein hoher Anteil an MDCS (entspricht einem hohen freien Kohlenstoffanteil im Temperaturbereich von 610 bis 650°C) mit dem klassifizierten Si_{mg} zu erwarten, kann es bevorzugt sein, das Si_{mg} der generell bei niedrigeren Temperaturen ablaufenden Hydrochlorierung zuzuführen.

Neben der Entscheidung, ob ein zu verarbeitendes Si_{mg} überhaupt der Chlorsilanherstellung zugeführt werden soll, kann somit auch zwischen verschiedenen Verfahren zur Chlorsilanherstellung differenziert werden. Ferner kann auf die zu erwartenden Mengen an Nebenprodukten geschlossen werden, wodurch sich die Reinigung der Chlorsilane optimieren lässt.

Vorzugsweise liegt das Si_{mg} für das erfindungsgemäße Verfahren in einer Partikelgröße von 1 bis 1000 µm, bevorzugt 50 bis 500 µm, besonders bevorzugt 100 bis 200 µm, vor.

### Bestimmen des freien Kohlenstoffanteils einer Si_{mg}-Probe:

Die Ergebnisse aus der Bestimmung des freien Kohlenstoffanteils ermöglichen die Identifizierung und damit die Selektion von besonders vorteilhaftem Si_{mg}-Ausgangsmaterial für die Synthese von Chlorsilanen, insbesondere von TCS. Der besondere Vorteil des erfindungsgemäßen Verfahrens besteht in einer Vermeidung der Bildung von kohlenstoffhaltigen Nebenprodukten und damit einhergehend in einer verminderten Belastung für die der Chlorsilanherstellung nachgeschalteten Destillationsprozesse. Mit besonderem Vorteil ermöglicht das Verfahren, dass auch stark mit anorganischem Kohlenstoff (der bei Temperaturen > 700°C mit O₂ reagiert) verunreinigtes Si_{mg} für die Chlorsilanherstellung selektiert werden kann, ohne eine verstärkte Bildung von Nebenprodukten befürchten zu müssen. So können beispielsweise Si_{mg}-Chargen, die bisher ausschließlich aufgrund ihres zu hohen Gesamtkohlenstoffgehalts von der Chlorsilanherstellung ausgeschlossen wurden, dennoch für die Chlorsilanherstellung verwendet werden, wenn mit dem erfindungsgemäßen Verfahren festgestellt wird, dass der freie Kohlenstoffgehalt lediglich bei oder unterhalb von 150 ppmw liegt (vgl. Tabelle 4). Zudem kann die Bestimmung des Gesamtkohlenstoffgehalts im betrieblichen Monitoring entfallen, d.h. es kann auf ein zweites Analysegerät wie den LECO C-200 verzichtet werden. Auch kann die früher üblicherweise durchgeführte und aufwändige Analyse kohlenstoffhaltiger Verbindungen auf der Si_{mg}-Oberfläche mittels Extraktion, gefolgt von NMR- und IR-spektroskopischen Messungen, entfallen.

Zur Bestimmung des freien Kohlenstoffgehalts wird vorzugsweise der Kohlenstoffanalysator LECO RC-612 verwendet. Mit dem LECO RC-612 ist es systembedingt nicht möglich den Gesamtkohlenstoff sondern nur den Oberflächenkohlenstoff zu bestimmen. Grundsätzlich wird beim LECO RC-612 die reine Si_{mg}-Probe (ohne Zuschlagstoffe) erhitzt, wobei nur der an der Oberfläche der Si_{mg}-Partikel befindliche Kohlenstoff im O₂-strom verbrannt und das entstandene CO₂ mittels IR-Messzelle quantitativ erfasst wird.

Bei der Bestimmung des Gehalts an Oberflächenkohlenstoff (oder auch des Gehalts an freiem Kohlenstoff) werden die bei den gewählten Messtemperaturen reaktiven Oberflächenkohlenstoffkontaminationen im O₂-Strom vollständig zu CO₂ oxidiert. O₂ dient dabei sowohl als Träger- als auch als Brenngas. Die CO₂-Konzentration im so erhaltenen Messgas wird standardmäßig mittels der im Analysegerät integrierten IR-Durchflussmesszellen bestimmt. Das Ergebnis wird als Massenanteil Oberflächenkohlenstoff (oder freier Kohlenstoff) bezogen auf die Probenmasse errechnet.

Das Analysegerät ist üblicherweise zusätzlich mit einer Einheit zur O₂-Vorreinigung ausgestattet. Diese Einheit oxidiert mögliche Spuren von Kohlenwasserstoffen im O₂ vor dem Kontakt mit der Si_{mg}-Probe katalytisch mit Kupferoxid bei 600°C zu CO₂ und entfernt das gebildete CO₂ sowie gegebenenfalls vorhandenes Wasser mittels geeigneter Absorptionsmedien (bspw. Magnesiumperchlorat und Natriumhydroxid) vollständig aus dem O₂. Diese vorreinigung erlaubt den Einsatz von technischem O₂ mit einer Reinheit von > 99,5% (Qualität O₂ 2.5). Versuche mit reinerem O₂ der Qualität 5.0 (> 99,999%) führen generell zu keinem besseren Ergebnis.

Nach der Vorreinigung wird der O₂ in ein horizontal angeordnetes, beheiztes Quarzrohr geführt. Das Quarzrohr bildet ein zur Atmosphäre offenes System. Das Eindringen von Luft wird durch einen ersten permanenten Spülstrom aus vorgereinigtem O₂ unterbunden. Ein zweiter Strom aus vorgereinigtem O₂ wird in zum ersten Strom entgegengesetzter Richtung geführt und streicht an der Messposition über die in einem Probenschiffchen aus Quarz angeordnete Si_{mg}-Probe. An der Messposition herrscht die gewählte Messtemperatur, bei welcher der Oberflächenkohlenstoff der Si_{mg}-Probe bestimmt werden soll.

Das Messgas, angereichert mit den Oxidationsprodukten der Si_{mg}-Probe, wird nach dem Verlassen des Quarzrohrs durch eine Nachbrennkammer bei 850°C geführt und anschließend an einem Kupferoxid-Katalysator bei 750°C vollständig zu CO₂ oxidiert. Der CO₂-Gehalt im Messgas wird standardmäßig mittels IRspektroskopie an zwei Durchflussmesszellen mit Längen von 17,78 mm (0,7 inches; High-Zelle) sowie 152,4 mm (6 inches; Low-Zelle) bei ∼2349 cm⁻¹ bestimmt. Bevor das Messgas über einen Kamin das Analysegerät verlässt, wird es durch Absorptionsmedien, die auch in der O₂-vorreinigung Verwendung finden, von CO₂ und Wasser befreit. Die Kalibrierung der beiden IR-Zellen erfolgt mittels Calciumcarbonat-Standards bei der High-Zelle und eines wässrigen Mannit-Standards bei der Low-Zelle. Die Kalibrierung der Zellen erfolgt bei Messbedingungen.

Die analytische Nachweisgrenze wird aus Messergebnissen von Blindwertmessungen eines Messtages nach der Leerwertmethode durch ein SPC-System (Statistical Process Control) errechnet. Bei Abweichungen erhält der Anwender eine Warnung, damit Maßnahmen zur Einhaltung der definierten Grenzen eingeleitet werden können.

Zur Vermeidung von Messfehlern wird der Messvorgang vorzugsweise in einer Laminar-Flow-Box der Reinheitsklasse 7 (10.000; nach ISO 14644-1) durchgeführt. Die Laminar-Flow-Box kann ferner in einem Reinraum der Reinheitsklasse 8 (100.000; nach ISO 14644-1) angeordnet sein.

Der Kohlenstoffanalysator LECO RC-612 ist in der Lage, sowohl bei einer konstanten Messtemperatur (statische Messmethode) als auch mit einer definierten Temperaturrampe (dynamische Messmethode) zwischen 60 und 120 K*min⁻¹ im Bereich von 100 bis 1100°C den Oberflächenkohlenstoff zu messen. Die statische Messung zielt insbesondere auf eine quantitative Bestimmung des gesamten Oberflächenkohlenstoffs ab. Die dynamische Messung zielt insbesondere auf eine qualitative Unterscheidung verschiedener Kohlenstoffspezies auf der Oberfläche des Si_{mg} ab. Die Messbedingungen der beiden Messmethoden sind in der Tabelle 1 zusammengefasst.

**Tabelle 1**

| | statische Messmethode | dynamische Messmethode |
|---|---|---|
| O₂-Fluss Spülgas [L*min⁻¹] | 4,0 | 4,0 |
| O₂-Fluss Reaktions- und Trägergas über die Probe [L*min⁻¹] | 0,75 | 0,40 |
| Messzeit [s] | 55 | 120 |
| Probeneinwaage [g] | 2,5 | 2,5 |
| Messtemperatur [°C] | 1000 (C[oberfl.]) und 700 (C[frei]) | 100-1000 (C[oberf1.]) und 100-700 (C[frei]) |
| Temperaturgradient [K*min⁻¹] | 0 | 120 |
| Analytische Nachweisgrenze für Kohlenstoff | 20 ppbw | 80 ppbw |

Das Degradationsverhalten von Kohlenstoffverbindungen, wie bspw. von Kohlenwasserstoffen oder von Polymeren, ist generell von Faktoren wie der Anwesenheit und Konzentration eines Oxidationsmittels (z.B. oxidative Degradation durch O₂), Hitzeeinwirkung (thermale Degradation), Lichteinwirkung (z.B. Photodegradation durch UV-Licht), des chemischen Aufbaus (Struktur, Vernetzungsgrad, Sättigung, Kristallinität, Formulierung) und der Beimengungen von Füllstoffen, Polymerisationskatalysatoren, Stabilisatoren, Inhibitoren und brandhemmenden Additiven abhängig. Bei der dynamischen Messmethode wird die temperaturabhängige thermooxidative Degradation von Kohlenstoffverbindungen als Prinzip zur Unterscheidung von Kohlenstoffspezies auf der Si_{mg}-Oberfläche ausgenutzt. Unterschiedliche Kohlenstoffspezies ergeben charakteristische Thermogramme. Die Kriterien zur Unterscheidung von Kohlenstoffspezies mittels Thermogrammen sind die Initialtemperatur (Beginn des thermooxidativen Abbaus) und die Kurvenform (Steigung, Lage und Anzahl der Maxima). Die Unterscheidung der Kohlenstoffspezies nach Stoffen oder Stoffklassen erfolgt üblicherweise durch Vergleiche mit Referenz-Thermogrammen.

Ein weiterer Aspekt der Erfindung ist ein Verfahren zur Herstellung von Chlorsilanen, insbesondere TCS, umfassend die Schritte
a) Bestimmen des freien Kohlenstoffanteils von metallurgischem Silicium, der bis zu einer Temperatur von 700°C mit Sauerstoff reagiert,
b) Bereitstellen von metallurgischem Silicium, bei dem der freie Kohlenstoffanteil ≤ 150 ppmw beträgt.
c) Umsetzen einer partikulären Kontaktmasse enthaltend das metallurgische Silicium aus Schritt b) mit einem Chlorwasserstoff enthaltenden Reaktionsgas.

Vorzugsweise weist das Si_{mg} einen Gesamtkohlenstoffgehalt von bis zu 2500 ppmw. Insbesondere weist das Si_{mg} für das Verfahren einen Gesamtkohlenstoffgehalt von 400 bis 2500 ppmw auf.

Chargen von Si_{mg}, deren Gesamtkohlenstoffgehalt derart hoch ist, wurden bisher üblicherweise nicht der Chlorsilanherstellung zugeführt. Durch das erfindungsgemäße Selektionsverfahren können solche Chargen jedoch für die Chlorsilanherstellung genutzt werden. Dadurch können insbesondere Kosten eingespart werden, da der Preis von Si_{mg} mit höherer Reinheit steigt.

Bei den Verfahren handelt es sich vorzugsweise um eine Hydrochlorierung oder eine NTK.

Die Hydrochlorierung läuft vorzugsweise in einem Temperaturbereich von 280 bis 400°C, besonders bevorzugt 320 bis 380°C, insbesondere 340 bis 360°C, ab. Die NTK läuft vorzugsweise in einem Temperaturbereich von 350 bis 850°C, besonders bevorzugt 400 bis 750°C, insbesondere 500 bis 700°C, ab.

Insbesondere handelt es sich bei den hergestellten Chlorsilanen um Chlorsilane der allgemeinen Formel HₙSiCl₄₋ₙ und/oder HₘCl₆₋ₘSi₂ mit n = 1 bis 3 und m = 0 bis 4. Vorzugsweise sind die Chlorsilane ausgewählt aus der Gruppe mit TCS, Dichlorsilan, Monochlorsilan, Si₂Cl₆, HSi₂Cl₅ und Mischungen daraus. Besonders bevorzugt handelt es sich um TCS.

Bei einem Verfahren zur Herstellung von Methylchlorsilanen kann es sich z.B. um eine Müller-Rochow-Synthese handeln, die üblicherweise bei einer Temperatur von 250 bis 350°C und einem Druck von 0,1 bis 0,5 MPa abläuft. Bei den dabei hergestellten Methylchlorsilanen kann es sich beispielsweise um Trimethylchlorsilan, Dimethyldichlorsilan, Dimethylchlorsilan, MDCS, MTCS und Mischungen daraus handeln.

Das beschriebene Verfahren kann in einen Verbund zur Herstellung von polykristallinem Silicium eingebunden sein. Der Verbund kann folgende Prozesse umfassen:
- Klassifizierung von Si_{mg} und Zuordnung für die Chlorsilanherstellung gemäß dem erfindungsgemäßen Verfahren,
- Erzeugung von Chlorsilanen, insbesondere TCS, mittels Hydrochlorierung oder NTK,
- Aufreinigung der erzeugten Chlorsilane zu Chlorsilanen TCS mit Halbleiterqualität,
- Abscheidung von polykristallinem Silicium, bevorzugt nach dem Siemens-Verfahren oder dem Granulatverfahren.

Beschrieben wird ferner auch eine Verwendung von Si_{mg} mit einem Gesamtkohlenstoffgehalt von bis zu 2500 ppmw, bevorzugt bis zu 1500 ppmw, besonders bevorzugt bis zu 750 ppmw, zur Herstellung von Chlorsilanen, insbesondere TCS, wobei der Anteil an freiem Kohlenstoff maximal 150 ppmw beträgt.

Das Si_{mg} kann mit einem Gesamtkohlenstoffgehalt von 400 bis 2500 ppmw zur Chlorsilanherstellung verwendet werden.

### BEISPIELE

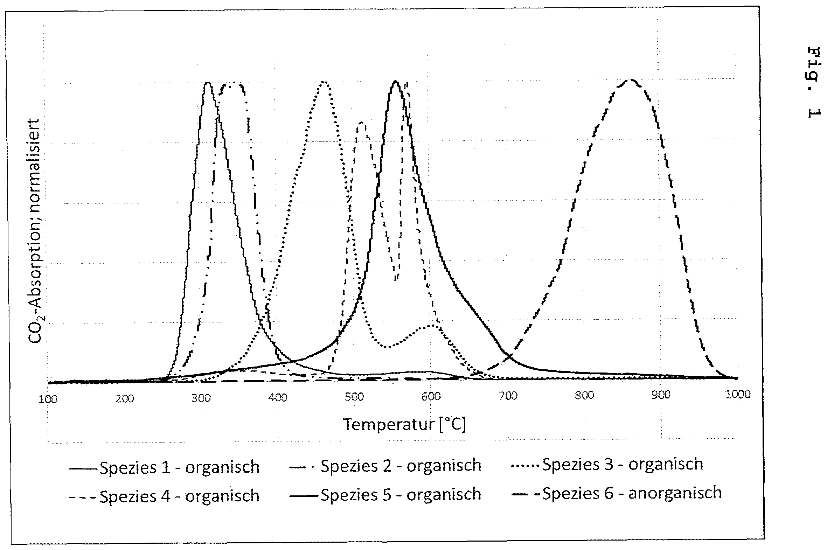

**Fig. 1** zeigt beispielhaft ein solches Referenz-Thermogramm, wobei jede Kurve einer Si_{mg}-Probe entspricht, die mit einem bekannten organischen polymeren Reinstoff kontaminiert ist (Spezies 1 bis 5 - organisch) bzw. typischen anorganischen Kohlenstoffspezies wie Carbonaten, Carbiden und allotropen Formen des Kohlenstoffs (Spezies 6 - anorganisch). Während die thermo-oxidative Degradation der organischen Kohlenstoffspezies im Temperaturbereich von ca. 250 und 750°C stattfindet, liegt die Initialtemperatur des thermo-oxidativen Abbaus der anorganischen Kohlenstoffverbindungen bei ca. 650°C. Die in den IR-Zellen gemessene CO₂-Absorption ist direkt proportional zur CO₂-Konzentration und wurde auf den Wert Eins normalisiert (d.h., der höchste Messwert ist jeweils auf Eins normalisiert).

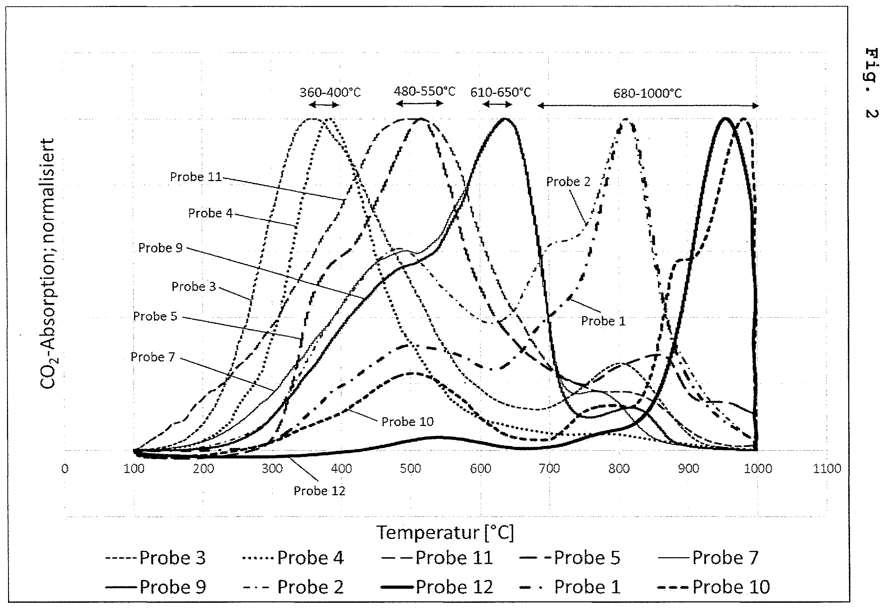

**Fig. 2** zeigt Thermogramme von zehn Proben aus zehn verschiedenen Chargen Si_{mg} (Proben 1 bis 5, 7, 9, 10 bis 12) in einem Messbereich von 100 bis 1000°C. Die Thermogramme zeigen sowohl monomodale (z.B. Probe 4) als auch multimodale (z.B. Proben 9, 10) Kurvenformen. Aus einem monomodalen Kurvenverlauf kann gefolgert werden, dass die betreffende Probe überwiegend mit nur einer kohlenstoffhaltigen Substanz oder mit unterschiedlichen Substanzen, die sehr ähnliche Oxidationseigenschaften aufweisen (z.B. identische Substanzklassen), kontaminiert ist. Der überwiegende Teil der Proben zeigt jedoch multimodale Thermogramme, aus denen gefolgert werden kann, dass diese Proben mit mehreren, in ihren Oxidationseigenschaften unterschiedlichen, kohlenstoffhaltigen Substanzen kontaminiert sind. Die integrale Fläche eines Thermogramms ist direkt proportional zu dem Gehalt an Kohlenstoff. Folglich ist die integrale Fläche unterhalb eines Peaks direkt proportional zu dem Anteil einer bestimmten kohlenstoff-haltigen Substanz oder Substanzklasse.

Es hat sich herausgestellt, dass es im Temperaturbereich der thermooxidativen Degradation von organischen Kohlenstoffspezies drei charakteristische Temperaturbereiche gibt, in welchen die gemessene Menge an freiem Kohlenstoff jeweils mit zumindest einem Nebenprodukt bei der Chlorsilanherstellung korreliert:
Temperaturbereich 1: 360 - 400°C
Temperaturbereich 2: 480 - 550°C
Temperaturbereich 3: 610 - 650°C

Anorganische Kohlenstoffspezies zeigen in den Thermogrammen der Proben ähnliche Gruppierungen ab einer Temperatur von > 680°C (Temperaturbereich 4: 680 - 1000°C).

Um festzustellen, ob die den Temperaturbereichen 1 bis 3 entsprechenden kohlenstoffhaltigen Verunreinigungen der Entstehung bestimmter Nebenprodukte bei Verfahren zur Herstellung von Chlorsilanen zugeordnet werden können, wurden die untersuchten Si_{mg}-Chargen bei der Hydrochlorierung und der NTK zur TCS-Herstellung umgesetzt. Das den Versuchsreaktor verlassende Gas wurde vollständig kondensiert und mittels Gaschromatographie quantitativ und qualitativ untersucht. Dabei wurden Kohlenstoffverbindungen (Nebenprodukte) wie bspw. iso-Pentan, MDCS und MTCS in unterschiedlichen Konzentrationen nachgewiesen. Eine Analyse der Daten ergab überraschenderweise einen Zusammenhang zwischen den gebildeten Nebenprodukten (in Art und Konzentration) und den in den Temperaturbereichen 1 bis 3 auftretenden Signalen.
1. Der Gehalt an iso-Pentan im Kondensat nach der TCS-Herstellung ist direkt proportional zum gemessenen Anteil an kohlenstoffhaltiger Verunreinigung (Fläche unterhalb des Signals) im Temperaturbereich 1.
2. Der Gehalt an MTCS im Kondensat nach der TCS-Herstellung ist direkt proportional zum gemessenen Anteil an kohlenstoffhaltiger Verunreinigung (Fläche unterhalb des Signals) im Temperaturbereich 2.
3. Der Gehalt an MDCS im Kondensat nach der TCS-Herstellung ist direkt proportional zum gemessenen Anteil an kohlenstoffhaltiger Verunreinigung (Fläche unterhalb des Signals) im Temperaturbereich 3.
4. Die bei der dynamischen Oberflächenkohlenstoffmessung erhaltenen Signale bei Temperaturen > 680°C (Temperaturbereich 4) zeigen keinerlei Korrelationen in Bezug auf die Bildung eines Nebenprodukts bei der TCS-Herstellung. Diese Kohlenstoffspezies sind inert bei den vorherrschenden Reaktionsbedingungen der Hydrochlorierung und NTK.

Beispielhaft ist dies für die Si_{mg}-Proben 4, 6, 8 und 12 gezeigt.

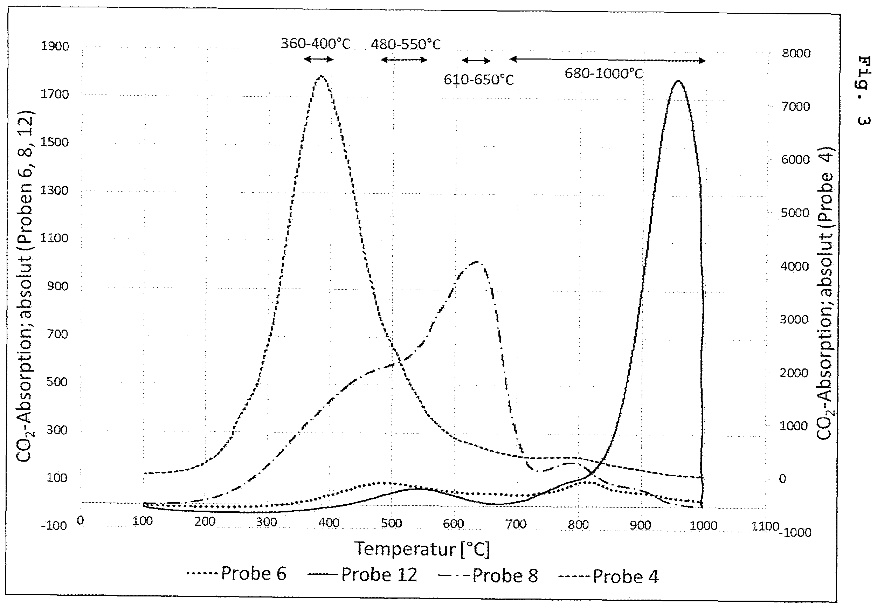

Die Si_{mg}-Proben 4, 6, 8 und 12 wurden den oben ausgeführten Messvorschriften unterzogen, um den Oberflächenkohlenstoff (Temperatur 100-1000°C) sowie den freien Kohlenstoff (Temperatur 100-700°C) zu bestimmen. **Fig. 3** zeigt die erhaltenen Thermogramme. Die CO₂-Absorption ist als absoluter Wert (realer Messwert der IR-Messzellen) angegeben.

Die den Proben 4, 6, 8 und 12 zugehörigen Si_{mg}-Chargen wurden bei der Hydrochlorierung umgesetzt und die Mengen an *iso-*Pentan, MDCS und MTCS im Produktkondensat ermittelt. Die Hydrochlorierung wurde für jede der vier Chargen in einem Wirbelschichtreaktor durchgeführt (vgl. US4092446, Fig. 12). Ein Bett aus Si_{mg} wird dabei mit Stickstoff bis zur Ausbildung einer Wirbelschicht gespült. Der Quotient aus Wirbelschichthöhe und Reaktordurchmesser liegt bei einem Wert von ca. 5. Die Temperatur der Wirbelschicht wird auf etwa 340°C eingestellt und wird mittels Kühlung in etwa konstant gehalten. Anschließend werden Chlorwasserstoff und Si_{mg} so zugegeben bzw. nachdosiert, dass die Höhe der Wirbelschicht über den Versuchszeitraum konstant bleibt und sich ein konstantes Molverhältnis der Reaktanden von 3:1 (HCl:Si_{mg}) einstellt. Der Druck im Reaktor beträgt üblicherweise 0,1 MPa (1 bar, Überdruck). Nach 48 h Laufzeit wird eine Flüssigkeitsprobe (Kondensat) entnommen. Die kondensierbaren Anteile des Produktgasstroms werden über eine Kühlfalle bei -40°C kondensiert und die anfallende Flüssigkeit mittels Gaschromatographie analysiert, wobei die Menge an kohlenstoffhaltigen Nebenprodukten bestimmt wird. Die Detektion erfolgt über einen Wärmeleitfähigkeitsdetektor. Aus den Analysenergebnissen zweier nach 48 h entnommener Proben werden jeweils die Mittelwerte gebildet. Die Analyse der kohlenstoffhaltigen Nebenprodukte bei der NTK erfolgt auf analoge Weise.

Eine Bewertung der vier Chargen (Proben 4, 6, 8, 12) hinsichtlich ihrer Qualität für die Herstellung von Chlorsilanen erfolgte basierend auf Ergebnissen einer Vielzahl von Versuchen mit verschiedenen Si_{mg}-Chargen sowie einer Abschätzung der durch deren Einsatz hervorgerufenen Kosten für die destillative Reinigung des entsprechenden Produktgemisches zur Herstellung von hochreinem TCS.

Die erhaltenen Ergebnisse sind in den Tabellen 2 und 3 zusammengefasst.

**Tabelle 2**

| | c(iso-Pentan) [ppbw] | c(MTCS) [ppbw] | c(MDCS) [ppbw] | IR-Absorpt. Low-Zelle ; Temp.-bereich 1 | IR-Absorpt. Low-Zelle; Temp. - bereich 2 | IR-Absorpt. Low-Zelle; Temp.-bereich 3 |
|---|---|---|---|---|---|---|
| Probe 6 | 216 | 3107 | 2779 | 26,5 | 84,3 | 51,1 |
| Probe 12 | 174 | 5023 | 5018 | 0 | 58,7 | 19,0 |
| Probe 8 | 4013 | 16231 | 14923 | 380,2 | 611,7 | 1001,6 |
| Probe 4 | 57743 | 22386 | 12139 | 6263,0 | 2040,3 | 579, 0 |

**Tabelle 3**

| | c (Oberflächen-C) [ppmw] | Qualität (iso-Pentan) | Qualität (MTCS) | Qualität (MDCS) | Qualität Mittelwert |
|---|---|---|---|---|---|
| Probe 6 | 7 | 1 | 1 | 1 | 1, 0 |
| Probe 12 | 68 | 1 | 1 | 1 | 1,0 |
| Probe 8 | 62 | 3 | 3 | 3 | 3,0 |
| Probe 4 | 423 | 6 | 5 | 4 | 5,0 |

Ein Oberflächenkohlenstoffgehalt (c(Oberflächen-C)) von 68 ppmw (Probe 12) ließe eigentlich nur auf durchschnittliche Eigenschaften bezüglich der Bildung kohlenstoffhaltiger Nebenprodukte schließen. Da der Oberflächenkohlenstoff der Probe 12 gemäß dem Thermogramm aus Fig. 3 dem anorganischen Bereich oberhalb von etwa 700°C zuzuordnen ist, führt das entsprechende Si_{mg} jedoch nur zu einer sehr geringen Bildung von kohlenstoff-haltigen Nebenprodukten bei der TCS-Herstellung. Dies bestätigt, dass anorganische Kohlenstoffverbindungen bei den Reaktionsbedingungen der TCS-Synthese (Hydrochlorierung und NTK) nicht zu störenden Nebenprodukten umgesetzt werden und als inert angesehen werden können.

Die Probe 8 weist im Thermogramm (Fig. 3) starke Signale in den Temperaturbereichen 2 und 3 auf, die mit der Bildung von MTCS und MDCS korrelieren. Beim Oberflächenkohlenstoff handelt es sich also hauptsächlich freien Kohlenstoff. Die Probe 8 liegt mit ihrer Menge an Oberflächenkohlenstoff von 62 ppmw noch im akzeptablen Bereich für die TCS-Synthese (Qualitätsstufe 3).

Die Probe 4 weist ein sehr starkes Signal im Temperaturbereich 1 auf, der mit der Bildung von iso-Pentan korreliert, was mit der GC-Analyse am Ende der TCS-Herstellung bestätigt wird (c(iso-Pentan) = 57743 ppbw). Insgesamt ergibt sich eine Menge an Oberflächenkohlenstoff von 423 ppmw. Da es sich hauptsächlich um freien Kohlenstoff handelt, führt die der Probe 4 zugrundeliegende Si_{mg}-Charge zu einem mangelhaften Gesamtergebnis (Qualitätsstufe 5). Derartige Chargen werden daher der Herstellung von Methylchlorsilanen zugeordnet. Es ist zu beachten, dass für die Probe 4 in der Fig. 3 die Sekundärachse (rechts) maßgeblich ist. Die Proben 4 und 8 zeigen nur sehr geringe Signale oberhalb von 700°C, weshalb die C-Gehalte in diesem Bereich vernachlässigbar sind. Ein Bewertung gemäß Tabelle 4 (Messbereich 100 bis 700°C) ist daher möglich.

Die Probe 6 weist einen sehr geringen Oberflächenkohlenstoffgehalt von 7 ppmw auf. Das Thermogramm der Probe 6 ähnelt im Bereich zwischen 100 und 700°C in seinem Verlauf dem der Probe 12. Es sind nur sehr geringe Mengen an iso-Pentan MTCS und MDCS zu erwarten, was sich durch die GC-Analyse am Ende der TCS-Herstellung bestätigt.

Da letztlich nur der Gehalt an freiem Kohlenstoff (Reaktion mit O₂ bis 700°C) über die Qualität des Si_{mg} hinsichtlich der Bildung unerwünschter Nebenprodukte bei der Herstellung von Chlorsilanen und damit über dessen Einsatzfähigkeit entscheidet, können Messungen im Regelbetrieb auch nur bei 700°C (statisch) bzw. in einem Temperaturbereich von 100 bis 700°C (dynamisch) erfolgen. Anhand des bei diesen Temperaturen bestimmten freien Kohlenstoffs kann die Einteilung in die Qualitätsstufen erfolgen (vgl. Tabelle 4), wobei Si_{mg}-Chargen in den Qualitätsstufen 5 und 6 generell einem Verfahren zur Herstellung von Methylchlorsilanen zugeordnet werden.

**Tabelle 4**

| Oberflächenkohlenstoffkonzentration [ppmw] bis 700°C | Qualitätsstufe |
|---|---|
| < 10 | 1 |
| > 10 - 30 | 2 |
| > 30 - 80 | 3 |
| > 80 - 150 | 4 |
| > 150 - 300 | 5 |
| > 300 | 6 |

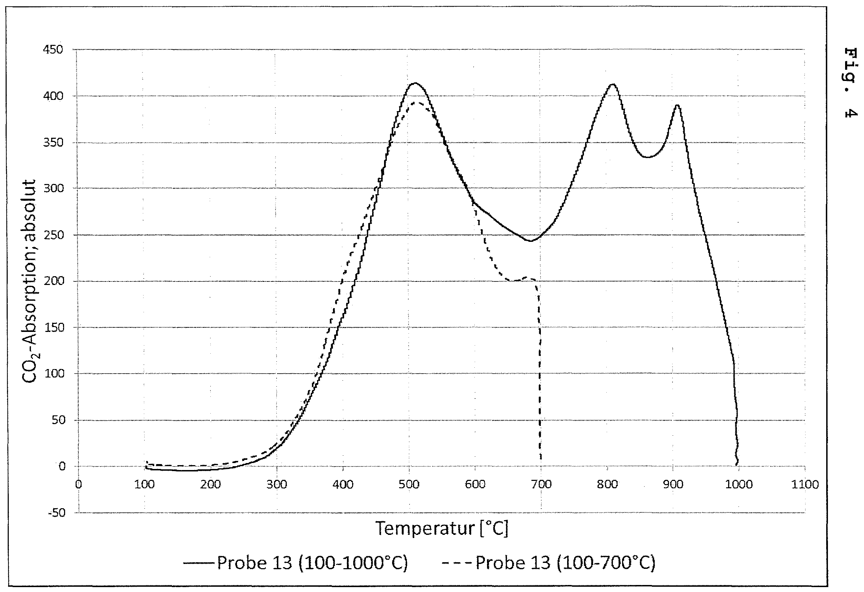

Die dynamische Messung ist dann angezeigt, wenn eine Bewertung hinsichtlich des ungefähren Mengenverhältnisses an verschiedenen Nebenprodukten erfolgen soll. Eine vergleichende Darstellung zwischen einer dynamischen Messung einer Probe Si_{mg} (Probe 13) bis 1000°C und bis 700°C ist in der **Fig. 4** gezeigt.

## Patentansprüche

1. Verfahren zur Klassifizierung von metallurgischem Silicium, das Verunreinigungen aus Kohlenstoff und/oder kohlenstoffhaltigen Verbindungen enthält, umfassend die Schritte
a) Bestimmen des freien Kohlenstoffanteils, der bis zu einer Temperatur von 700°C mit Sauerstoff reagiert,
b) Zuordnen von metallurgischem Silicium, bei dem der freie Kohlenstoffanteil ≤ 150 ppmw beträgt, für ein Verfahren zur Herstellung von Chlorsilanen und/oder Zuordnen von metallurgischem Silicium, bei dem der freie Kohlenstoffanteil > 150 ppmw beträgt, für ein Verfahren zur Herstellung von Methylchlorsilanen.

2. Verfahren nach Anspruch 1, **dadurch gekennzeichnet, dass** metallurgisches Silicium, bei dem der freie Kohlenstoffanteil ≤ 80 ppmw, bevorzugt ≤ 30 ppmw, besonders bevorzugt ≤ 10 ppmw beträgt, dem Verfahren zur Herstellung von Chlorsilanen und/oder metallurgisches Silicium, bei dem der freie Kohlenstoffanteil > 80 ppmw, bevorzugt > 30 ppmw, besonders bevorzugt > 10 ppmw, beträgt, dem Verfahren zur Herstellung von Methylchlorsilanen zugeordnet wird.

3. Verfahren nach Anspruch 1 oder 2, **dadurch gekennzeichnet, dass** alternativ oder zusätzlich die Bestimmung des freien Kohlenstoffanteils temperaturfraktioniert für mindestens einen der Temperaturbereiche von 360 bis 400°C, von 480° bis 550°C und von 610 bis 650°C erfolgt, wobei der im Temperaturbereich von 360 bis 400°C bestimmte freie Kohlenstoffanteil mit einer Menge an *iso*-Pentan, der im Temperaturbereich von 480 bis 550°C bestimmte freie Kohlenstoffanteil mit einer Menge an Methyltrichlorsilan und der im Temperaturbereich von 610 bis 650°C bestimmte freie Kohlenstoffanteil mit einer Menge an Methyldichlorsilan korreliert und in Abhängigkeit der Korrelation das metallurgische Silicium hinsichtlich seiner Qualität bewertet wird.

4. Verfahren nach Anspruch 3, **dadurch gekennzeichnet, dass** in Abhängigkeit der Korrelation das metallurgische Silicium unterschiedlichen Verfahren zur Herstellung von Chlorsilanen zugeordnet wird.

5. Verfahren nach einem der vorhergehenden Ansprüche, **dadurch gekennzeichnet, dass** das metallurgische Silicium in einer Partikelgröße von 1 bis 1000 µm, bevorzugt 50 bis 500 µm, besonders bevorzugt, 100 bis 200 µm, vorliegt.

6. Verfahren zur Herstellung von Chlorsilanen, umfassend die Schritte
a) Bestimmen des freien Kohlenstoffanteils von metallurgischem Silicium, der bis zu einer Temperatur von 700°C mit Sauerstoff reagiert,
b) Bereitstellen von metallurgischem Silicium, bei dem der freie Kohlenstoffanteil ≤ 150 ppmw beträgt.
c) Umsetzen einer partikulären Kontaktmasse enthaltend das metallurgisches Silicium aus Schritt b) mit einem Chlorwasserstoff enthaltenden Reaktionsgas.

7. Verfahren nach Anspruch 6, **dadurch gekennzeichnet, dass** das metallurgische Silicium eine Gesamtkohlenstoffgehalt von bis zu 2500 ppmw aufweist.

8. Verfahren nach Anspruch 6, **dadurch gekennzeichnet, dass** das metallurgische Silicium einen Gesamtkohlenstoffgehalt von 400 bis zu 2500 ppmw aufweist.

9. Verfahren nach einem der Ansprüche 6 bis 8, **dadurch gekennzeichnet, dass** es sich bei dem Verfahren zur Herstellung von Chlorsilanen um eine Hydrochlorierung und/oder eine Niedertemperaturkonvertierung handelt.

10. Verfahren nach einem der Ansprüche 6 bis 9, **dadurch gekennzeichnet, dass** es sich um Chlorsilane der allgemeinen Formel HₙSiCl₄₋ₙ und/oder HₘCl₆₋ₘSi₂ mit n = 1 - 3 und m = 0-4 handelt.

11. Verfahren nach einem der Ansprüche 6 bis 10, **dadurch gekennzeichnet, dass** die Chlorsilane ausgewählt sind aus der Gruppe mit Trichlorsilan, Dichlorsilan, Monochlorsilan, Si₂Cl₆, HSi₂Cl₅ und Mischungen daraus.

## Claims

1. Process for classification of metallurgical silicon containing impurities of carbon and/or carbon-containing compounds comprising the steps of
a) determining the free carbon proportion which reacts with oxygen up to a temperature of 700°C,
b) assigning metallurgical silicon in which the free carbon proportion is ≤ 150 ppmw for a process for producing chlorosilanes and/or assigning metallurgical silicon in which the free carbon proportion is > 150 ppmw for a process for producing methylchlorosilanes.

2. Process according to Claim 1, **characterized in that** metallurgical silicon in which the free carbon proportion is ≤ 80 ppmw, preferably ≤ 30 ppmw, particularly preferably ≤ 10 ppmw, is assigned to the process for producing chlorosilanes and/or metallurgical silicon in which the free carbon proportion is > 80 ppmw, preferably > 30 ppmw, particularly preferably > 10 ppmw, is assigned to the process for producing methylchlorosilanes.

3. Process according to Claim 1 or 2, **characterized in that** alternatively or in addition the determination of the free carbon proportion is carried out in temperature-fractionated fashion for at least one of the temperature ranges from 360°C to 400°C, from 480°C to 550°C and from 610°C to 650°C, wherein the free carbon proportion determined in the temperature range from 360°C to 400°C is correlated with an amount of isopentane, the free carbon proportion determined in the temperature range from 480°C to 550°C is correlated with an amount of methyltrichlorosilane and the free carbon proportion determined in the temperature range from 610°C to 650°C is correlated with an amount of methyldichlorosilane and the metallurgical silicon is evaluated in terms of its quality according to the correlation.

4. Process according to Claim 3, **characterized in that** the metallurgical silicon is assigned to different processes for producing chlorosilanes according to the correlation.

5. Process according to any of the preceding claims, **characterized in that** the metallurgical silicon has a particle size of 1 to 1000 µm, preferably 50 to 500 µm, particularly preferably 100 to 200 µm.

6. Process for producing chlorosilanes comprising the steps of
a) determining the free carbon proportion of metallurgical silicon which reacts with oxygen up to a temperature of 700°C,
b) providing metallurgical silicon having a free carbon proportion ≤ 150 ppmw,
c) reacting a particulate contact mass containing the metallurgical silicon from step b) with a hydrogen chloride-containing reaction gas.

7. Process according to Claim 6, **characterized in that** the metallurgical silicon has a total carbon content of up to 2500 ppmw.

8. Process according to Claim 6, **characterized in that** the metallurgical silicon has a total carbon content of 400 to 2500 ppmw.

9. Process according to any of Claims 6 to 8, **characterized in that** the process for producing chlorosilanes is a hydrochlorination and/or a low temperature conversion.

10. Process according to any of Claims 6 to 9, **characterized in that** chlorosilanes of general formula HₙSiCl₄₋ₙ and/or HₘCl₆₋ₘSi₂ where n = 1-3 and m = 0-4 are concerned.

11. Process according to any of Claims 6 to 10, **characterized in that** the chlorosilanes are selected from the group comprising trichlorosilane, dichlorosilane, monochlorosilane, Si₂Cl₆, HSi₂Cl₅ and mixtures thereof.

## Revendications

1. Procédé pour la classification de silicium métallurgique, qui contient des impuretés constituées par du carbone et/ou des composés carbonés, comprenant les étapes
a) détermination de la proportion de carbone libre qui réagit avec de l'oxygène jusqu'à une température de 700°C,
b) attribution du silicium métallurgique, dans lequel la proportion de carbone libre est ≤ 150 ppm en poids, à un procédé de préparation de chlorosilanes et/ou attribution du silicium métallurgique, dans lequel la proportion de carbone libre est > 150 ppm en poids, à un procédé pour la préparation de méthylchlorosilanes.

2. Procédé selon la revendication 1, **caractérisé en ce que** le silicium métallurgique, dans lequel la proportion de carbone libre est ≤ 80 ppm en poids, de préférence ≤ 30 ppm en poids, de manière particulièrement préférée ≤ 10 ppm en poids, est attribué au procédé pour la préparation de chlorosilanes et/ou le silicium métallurgique dans lequel la proportion de carbone libre est > 80 ppm en poids, de préférence > 30 ppm en poids, de manière particulièrement préférée > 10 ppm en poids, est attribué au procédé pour la préparation de méthylchlorosilanes.

3. Procédé selon la revendication 1 ou 2, **caractérisé en ce qu'**en variante ou en plus, la détermination de la proportion de carbone libre a lieu de manière fractionnée en ce qui concerne la température pour au moins l'une des plages de température de 360 à 400°C, de 480° à 550°C et de 610 à 650°C, la proportion de carbone libre déterminée dans la plage de température de 360 à 400°C étant corrélée à une quantité d'isopentane, la proportion de carbone libre déterminée dans la plage température de 480 à 550°C étant corrélée à une quantité de méthyltrichlorosilane et la proportion de carbone libre déterminée dans la plage de température 610 à 650°C étant corrélée à une quantité de méthyldichlorosilane et le silicium métallurgique étant évalué en ce qui concerne sa qualité en fonction de la corrélation.

4. Procédé selon la revendication 3, **caractérisé en ce que** le silicium métallurgique est attribué à différents procédés pour la préparation de chlorosilanes en fonction de la corrélation.

5. Procédé selon l'une quelconque des revendications précédentes, **caractérisé en ce que** le silicium métallurgique se trouve sous forme d'une grosseur de particule de 1 à 1000 µm, en particulier de 50 à 500 µm, de manière particulièrement préférée de 100 à 200 µm.

6. Procédé pour la préparation de chlorosilanes, comprenant les étapes
a) détermination de la proportion de carbone libre de silicium métallurgique qui réagit avec de l'oxygène jusqu'à une température de 700°C,
b) mise à disposition de silicium métallurgique dans lequel la proportion de carbone libre est ≤ 150 ppm en poids.
c) transformation d'une masse de contact particulaire contenant le silicium métallurgique provenant de l'étape b) avec un gaz réactionnel contenant de l'acide chlorhydrique.

7. Procédé selon la revendication 6, **caractérisé en ce que** le silicium métallurgique présente une teneur totale en carbone de jusqu'à 2500 ppm en poids.

8. Procédé selon la revendication 6, **caractérisé en ce que** le silicium métallurgique présente une teneur totale en carbone de 400 jusqu'à 2500 ppm en poids.

9. Procédé selon l'une quelconque des revendications 6 à 8, **caractérisé en ce qu'**il s'agit, pour le procédé de préparation de chlorosilanes, d'une hydrochloration et/ou d'une conversion basse température.

10. Procédé selon l'une quelconque des revendications 6 à 9, **caractérisé en ce qu'**il s'agit de chlorosilanes de formule générale HₙSiCl₄₋ₙ et/ou HₘCl₆₋ₘSi₂ dans lesquelles n = 1 - 3 et m = 0 - 4.

11. Procédé selon l'une quelconque des revendications 6 à 10, **caractérisé en ce que** les chlorosilanes sont choisis dans le groupe constitué par le trichlorosilane, le dichlorosilane, le monochlorosilane, Si₂Cl₆, HSi₂Cl₅ et leurs mélanges.
